# EUROPEAN PATENT APPLICATION

(11) **EP 1 510 582 A1**
(43) Date of publication of application: **02.03.2005**
(21) Application number: 04020085.9
(22) Date of filing: 24.08.2004
(51) Int. Cl.: C12N 15/52, C12N 1/21, C12N 9/02, C12N 9/10, C12P 13/04, C12P 13/10

(54) **Method for producing L-amino acid using bacterium of Enterobacteriaceae family, having nir operon inactivated**

(30) Priority: 29.08.2003 RU 2003126289
(71) Applicant: Ajinomoto Co., Inc., Tokyo (JP)
(72) Inventor: Ptitsyn, Leonid Romanovich, 115404 Moscow (RU); Altman, Irina Borisovna, 119435 Moscow (RU); Smirnov, Sergey Vasil'evich, 121552 Moscow (RU); Samsonova, Natalia Nikolaevna, 117186 Moscow (RU); Ermishev, Vladimir Yurievich, 127644 Moscow (RU)
(74) Representative: Strehl Schübel-Hopf & Partner

(57) **Abstract**

A method is provided for producing L-amino acid, such as L-arginine using a bacterium of *Enterobacteriaceae* family, particularly a bacterium belonging the genus *Escherichia*, with an inactivated *nir* operon.

## Description

### Background of the Invention

### Field of the Invention

The present invention relates to the microbiological industry, and specifically to a method for producing an L-amino acid using bacterium of *Enterobacteriaceae* family, wherein the *nir* operon, including the *nirBDC-cysG* genes, is inactivated.

### Description of the Related Art

*Escherichia coli* possesses two biochemically distinct nitrite reductase enzymes encoded by the *nrfABCDEFG* and *nirBDC* operons, respectively (Cole, J., FEMS Microbiol. Lett. 136:1-11 (1996)). A basal expression level of the *nir* operon is about 8 times higher than that of the *nrf* operon and can be increased 21-fold by adding nitrate (Wang, H. and Gunsalus, H.P., J. Bacteriol., 182, No. 20, p. 5813-5822 (2000)). Transcription of the *nirBDC* operon is driven from a single promoter, and expression is activated by two environmental signals: an absence of oxygen and a presence of nitrite or nitrate ions in the growth medium (Jayaraman et al., J. Mol. Biol., 196, 4:781-8 (1987); Page et al., Arch Microbiol., 154:4:349-54, (1990)). Also, the *cysG* gene is co-transcribed with the *nirBDC* operon, while the second constitutive promoter is located less than 100 bp upstream of the *cysG* gene (Peakman, T. et al, Eur. J. Biochem., 191(2):325-331 (1990)).

The product of the *cysG* gene, siroheme synthase, catalyzes the synthesis of a heme cofactor, siroheme, which is employed by sulfite reductase and nitrite reductase enzymes in the sulfate and nitrite reduction processes.

The NirBDC nitrite reductase is a siroheme-containing enzyme that uses NADH as an electron donor to reduce nitrite in the cytoplasm (MacDonald, H. and Cole, J., Mol. Gen. Genet., 200:320-334 (1985); Peakman, T. et al, Eur. J. Biochem., 191:315-323 (1990)). *E. coli* mutants defective in the *nirB* gene lack NADH-dependent nitrite reductase activity and reduce nitrite slowly during anaerobic growth. These mutants require cysteine for growth (Cole, J.A. et al, J. Gen. Microbiol. 120:475-483 (1980)).

The auxotrophy of *cysG* mutants is a result of their failure to produce siroheme, the cofactor of the CysIJ enzyme, sulfite reductase. Siroheme-dependent sulfite reduction is required for synthesis of cysteine, methionine, and other sulfur-containing metabolites whenever sulfate or sulfite is utilized as a sulfur source in the synthesis of these metabolites (Becker, M.A. et al, J. Biol. Chem., 244:2418-2427 (1969); Becker, M.A. and Tomkins, G.M., J. Biol. Chem., 244:6023-6030 (1969)). Since siroheme is also required for nitrite reductase (NirB), *cysG* mutants are also defective in reduction of nitrite. The *nir* promoter (Pₙᵢᵣ) contains an FNR binding site (position -41.5); a NarL/NarP binding site (position -69.5) (Jayaraman, P.S. et al, Nucleic Acids Res. 17:1 135-45 (1989); Tyson, K.L. et al, Mol. Microbiol., 7:1:151-7 (1993)); a Fis binding sites (-142, +23); an IHF binding site (-88); and a binding site for the nucleoid associated protein, H-NS, which preferentially binds to upstream sequences at the *nir* promoter.

The *nir* promoter is repressed by the following three DNA binding proteins: Fis, IHF and H-NS. The activation of *nir* promoter expression is co-dependent on both the FNR protein (an anaerobically triggered transcription activator) and the NarL or NarP proteins (transcription activators triggered by nitrite and nitrate). Under anaerobic conditions, FNR binds to a site -41.5, activating transcription of the *nir* operon. The *nir* promoter is further regulated by the presence of nitrite or nitrate ions in the medium. This is achieved by two very similar response-regulator family transcription factors, NarL and NarP (reviewed by Darwin, A.J. et al, Mol. Microbiol., 20:3:621-32 (1996)). In response to nitrite or nitrate, NarL and NarP are phosphorylated by the membrane-bound sensor kinase proteins, NarX and NarQ. Phosphorylated NarL and NarP then bind to specific heptamer sequences at target promoters and either up- or down-regulate transcription initiation at these promoters (for examples, see Tyson, K.L. et al., Mol. Microbiol., 13:6:1045-55 (1994); Darwin, A.J. et al., Mol. Microbiol., 25:3:583-95 (1997)).

The association of Fis, IHF and H-NS suggests that *nir* promoter DNA is sequestrated into a highly ordered nucleo-protein structure that represses FNR-dependent transcription activation. NarL and NarP can relieve both IHF- and Fis-mediated repression, but are unable to counteract H-NS mediated repression (Browning D.F. et al, Molecular Microbiology, 37(5), 1258-1269 (2000)).

The high nitrite conditions needed for *nirB* induction are consistent with the proposed role of the NirB enzyme in detoxification (Fazzio, T. G., and Roth, J. R., J. Bacteriol. 178:6952-6959 (1996)). A second plausible role for the NirB enzyme is thatit recycles NADH by oxidizing it in the presence of excess reducing equivalents. Such conditions occur when sufficient energy is generated by nitrate-dependent respiration via the NarG nitrate reductase complex. The presence of the NirB enzyme would thus allow the cell to effectively decouple carbon dissimilation from the nitrite respiratory pathways by using a futile cycle for NADH-NAD recycling (Wang, H. and Gunsalus, R.P., J. Bacteriol., 182, No. 20, p. 5813-5822 (2000)).

There have been no reports to date describing inactivation of the *nir* operon for the purpose of producing L-amino acids.

### SUMMARY OF THE INVENTION

An object of present invention is to enhance the productivity of L-amino acid producing strains. It is a further object of the invention to provide a method for producing L-amino acids using these strains.

It is a further object of the present invention to provide an L-amino acid- producing bacterium of the *Enterobacteriaceae* family, wherein the bacterium has been modified to inactivate the *nir* operon.

It is a further object of the present invention to provide the L-amino acid-producing bacterium as described above, wherein said *nir* operon comprises the *nirBDC* and *cysG* genes.

It is a further object of the present invention to provide the bacterium as described above, wherein the bacterium belongs to the genus *Escherichia.*

It is a further object of the present invention to provide the L-amino acid producing bacterium as described above, wherein said L-amino acid is L-arginine.

It is a further object of the present invention to provide the L-amino acid-producing bacterium as described above, wherein the bacterium has been modified to enhance expression of an L-arginine operon.

It is a further object of the present invention to provide a method for producing L-amino acid comprising:
- cultivating the bacterium as described above in a medium, and
- collecting the accumulated L-amino acid from the medium.

It is a further object of the present invention to provide the method as described above, wherein said L-amino acid is L-arginine.

It is a further object of the present invention to provide the method as described above, wherein the bacterium has been modified to enhance expression of an L-arginine operon.

### Brief Description of Drawings

Figure 1 shows the relative positions of the primers nirBL and nirBR on the plasmid pACYC184, which is used for amplification of *cat* gene.
Figure 2 shows the construction of the chromosomal DNA fragment, which contains an inactivated *nir* operon.

### DETAILED DESCRIPTION OF THE INVENTION

The aforementioned objects were achieved by finding that the inactivation of the *nir* operon can enhance production of L-amino acids such as L-arginine. Thus, the present invention has been completed.

The present invention is described in details below.

### 1. Bacterium of the present invention

The bacterium of the present invention is an L-amino acid-producing bacterium of *Enterobacteriaceae* family, wherein the bacterium has been modified to inactivate the *nir operon.*

In the present invention, "L-amino acid-producing bacterium means a bacterium, which has an ability to produce and cause accumulation of an L-amino acid in a medium, when the bacterium of the present invention is cultured in the medium. The L-amino acid-producing ability may be imparted or enhanced by breeding. The term "L-amino acid-producing bacterium" as used herein may also mean a bacterium which is able to produce and cause accumulation of L-amino acid in a culture medium in an amount larger than a wild-type or parental strain of bacterium, such as *E*. *coli* K-12 strain.

The bacterium of *Enterobacteriaceae* family that can be used in the present invention is not particularly limited, however, for example, bacteria described by Neidhardt, F.C. et al. (*Escherichia coli* and *Salmonella typhimurium,* American Society for Microbiology, Washington D.C., 1208, Table 1) are encompassed. Specifically, the *Enterobacteriaceae* family of bacteria includes bacteria belonging to the genera *Escherichia, Erwinia, Providencia* and *Serratia.* The genus *Escherichia* is preferred.

The phrase "a bacterium belonging to the genus *Escherichia"* means that the bacterium is classified as the genus *Escherichia* according to the classification known to a person skilled in the art of microbiology. A microorganism belonging to the genus *Escherichia* as used in the present invention inludes, but is not limited to, *Escherichia coli* (*E. coli*), which is most preferred bacterium for the present invention.

The phrase "*nir operon* is inactivated" or "to inactivate the *nir* operon" means that the target operon is modified in such a way that the modified gene of the operon encodes a mutant protein with decreased or no activity. It is also possible that the modified DNA region is unable to naturally express the operon due to the deletion of a part of the operon, shifting the reading frame of the operon gene(s), or the modification of adjacent regions of the operon, including sequences which control operon expression, such as promoter(s), enhancer(s), attenuator(s) etc.. The expression of the *nir* operon is driven from a single promoter located upstream of the *nirB* gene, and the constitutive basal expression level of the *cysG* gene is not sufficient for siroheme synthesis, due to its own weak promoter., It is possible, therefore, to inactivate only the *nirB* gene so that further expression of genes located downstream of the *nirB* gene becomes impossible. The role of nitrite reductase, encoded by the *nirB* gene, in L-arginine production remains unclear. One possible explanation is that inactivation of the *nirB* gene blocks transcription of the *cysG* gene from the *nirB* promoter and expression of the *cysG* gene from its own weak promoter is not sufficient for siroheme synthesis. This leads to a deficiency in the synthesis of cysteine, methionine and other sulfur-containing metabolites inducing the L-arginine biosynthetic pathway. So, one embodiment of the present invention includes inactivation or disruption of the *cysG* gene.

The *nir* operon of *E. coli* includes the following consecutively located genes: *nirB, nirD, nirC* and *cysG.* The *nirB* and *nirD* genes encode a nitrite reductase. The *nirC* gene encodes a nitrite transporter. The *cysG* gene encodes a siroheme synthase. The *nirB* gene (gi:16131244; numbers 3491648 to 3494191 in the GenBank accession number NC_000913.1), *nirD* gene (gi:16131245; numbers 3494188 to 3494514 in the GenBank accession number NC_000913.1), *nirC* gene (gi:16132233; numbers 3494640 to 3495446 in the GenBank accession number NC_000913.1) and *cysG* gene (gi:16131246; numbers 3495465 to 3496838 in the GenBank accession number NC_000913.1) are located between the *yhfC* and *yhfL* ORFs on the *E. coli* strain K-12 chromosome. The nucleotide sequence of the *nir* operon from *E. coli* MG1655 strain is registered in GenBank under accession No. AE000412 U00096. The nucleotide sequence comprising the *nirB, nirD, nirC* and *cysG* genes of the MG1655 strain is shown in SEQ ID NO: 6. The amino acid sequences encoded by the *nirB, nirD, nirC* and *cysG* genes are shown in SEQ ID NOS: 7, 8, 9 and 10, respectively. The coding regions of the *nirB, nirD, nirC* and *cysG* genes in the nucleotide sequence of SEQ ID NO: 6 are 135-2678, 2675-3001, 3379-3933 and 3952-5325, respectively.

Inactivation of the gene can be performed by conventional methods, such as mutagenesis treatment using UV irradiation or nitrosoguanidine (N-methyl-N'-nitro-N-nitrosoguanidine) treatment, site-directed mutagenesis, gene disruption using homologous recombination or/and insertion-deletion mutagenesis (Datsenko K.A. and Wanner B.L., Proc. Natl. Acad. Sci. USA, 2000, 97:12: 6640-45) which is also called "Red-driven integration".

The *nir* operon of a bacterium of *Enterobacteriaceae* family other than *E. coli* can also be inactivated by homologous recombination using the *nir* operon fragment from *E*. *coli* or a fragment of an inherent *nir* operon which may be a homologue to the *E. coli nir* operon. Such a *nir* operon homologue may have homology of not less than 70 %, preferably not less than 80 %, more preferably not less than 90%, and most preferably not less than 95% to the *E. coli nir* operon with respect to the nucleotide sequence of respective coding regions.

### L- arginine producing bacterium.

As a parent strain which is to be modified to inactivate the *nir* operon, L-arginine-producing bacteria are encompassed.

Bacteria belonging to the genus *Escherichia* which produce L-arginine include, but are not limited to, *E. coli* strain 237 (VKPM B-7925) and it's derivative strains harboring a mutant N-acetylglutamate synthase (Russian Patent No. 2215783), and an arginine-producing strain into which the *argA* gene encoding N-acetylglutamate synthetase is introduced (Japanese Laid-Open Publication No. 57-5693), and the like. The strain 237 is a mutant resistant to a pyrimidine analog, 6-azauracil, which was derived from *E. coli* K12 ilvA::Tn5 using N-methyl-N'-nitro-N-nitrosoguanidine (NTG). The strain 237 was deposited at Russian National Collection of Industrial Microorganisms (VKPM) on April 10, 2000, and received an accession number of VKPM B-7925, and was converted to an international deposit under the provisions of Budapest Treaty on May 18, 2001.

The bacterium of the present invention can be obtained by inactivation of *nir* operon in a bacterium which inherently has the ability to produce an L- amino acid. Alternatively, the bacterium of present invention can be obtained by imparting the ability to produce an L- amino acid to a bacterium which already has an inactivated *nir* operon.

Methods for preparation of plasmid DNA, digestion and ligation of DNA, transformation, selection of an oligonucleotide as a primer and the like may be ordinary methods well known to one skilled in the art. These methods are described, for instance, in Sambrook, J., Fritsch, E.F., and Maniatis, T., "Molecular Cloning A Laboratory Manual, Second Edition", Cold Spring Harbor Laboratory Press (1989).

### 2. Method of the present invention

The method of the present invention is a method for producing an L-amino acid having the following steps: cultivating the bacterium of the present invention in a culture medium which results in production accumulation of the L-amino acid in the medium, and collecting the accumulated L-amino acid from the medium. More specifically, the method of the present invention is a method for producing L-arginine, which method includes the steps of cultivating the bacterium of the present invention in a culture medium,, and collecting the accumulated L-arginine from the medium.

In the present invention, the cultivation, collection and purification of L-amino acid from the medium and the like may be performed by conventional fermentation methods typically used for production of an amino acid from a bacterium.

A medium used for the culture may be either synthetic or natural, so long as the medium includes a carbon source and a nitrogen source and minerals and, if necessary, appropriate amounts of nutrients which the bacterium requires for growth.

The carbon source may include various carbohydrates such as glucose and sucrose, and various organic acids. Depending on the mode of assimilation of the used microorganism, alcohol including ethanol and glycerol may be used.

As the nitrogen source, various ammonium salts such as ammonia and ammonium sulfate, other nitrogen compounds such as amines, a natural nitrogen source such as peptone, soybean-hydrolysate, and digested fermentative microorganism may be used.

As minerals, potassium monophosphate, magnesium sulfate, sodium chloride, ferrous sulfate, manganese sulfate, calcium chloride, and the like may be used. As vitamins, thiamine, yeast extract and the like may be used.

The cultivation is preferably performed under aerobic conditions such as a shaking culture, and stirring culture with aeration, at a temperature of 20 to 40 °C, preferably 30 to 38 °C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to the accumulation of the target L-amino acid in the liquid medium.

After cultivation, solids such as cells can be removed from the liquid medium by centrifugation or membrane filtration, and then the L-amino acid can be collected and purified by ion-exchange, concentration and/or crystallization methods.

### Examples

The present invention will be more concretely explained with reference to the following non-limiting Examples. In the Examples, arginine is of L-configuration.

### Example 1. Construction the strain having an inactivated nir operon.

### Deletion of the nirB gene.

Deletion of the *nirB* gene was performed by the method first developed by Datsenko and Wanner (Proc. Natl. Acad. Sci. USA, 2000, 97(12), 6640-6645) and called "Red-driven integration". According to this procedure, the PCR primers nirBL (SEQ ID NO: 1) and nirBR (SEQ ID NO: 2), which are homologous to both regions adjacent to the *nirB* gene, and a gene conferring antibiotic resistance in the template plasmid were constructed. Plasmid pACYC184 (NBL Gene Sciences Ltd., UK) (GenBank/EMBL accession number X06403) was used as a template in PCR reaction. PCR was conducted as follows: denaturation step for 3 min at 95 °C; profile for two first cycles: 1 min at 95 °C, 30 sec at 50 °C, 40 sec at 72 °C; profile for the last 25 cycles: 30 sec at 95 °C, 30 sec at 54 °C, 40 sec at 72 °C; final step: 5 min at 72 °C.

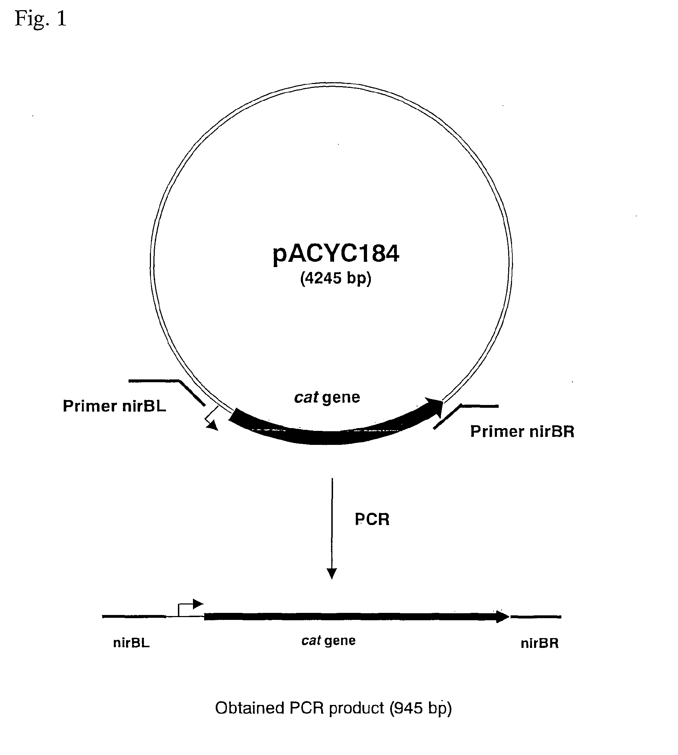

The obtained 945 bp PCR product (Fig. 1, SEQ ID NO: 3) was purified by agarose gel electrophoresis and used for electroporation of the *E*. *coli* strain MG1655 which harbors the plasmid pKD46 with temperature sensitive replication. The plasmid pKD46 (Datsenko and Wanner, Proc. Natl. Acad. Sci. USA, 2000, 97:12:6640-45) includes a 2,154 nucleotide (31088-33241) DNA fragment of phage λ (GenBank accession No. J02459), which contains the λ Red homologous recombination system genes (γ, β, exo genes) under the control of the arabinose-inducible P_{araB} promoter. The plasmid pKD46 is necessary for integration of the PCR product into MG1655 strain chromosome.

Electrocompetent cells were prepared as follows: overnight culture of *E*. *coli* strain MG1655 grown at 30 °C in LB medium supplemented with ampicillin (100 mg/l) was diluted 100 times with 5 ml of SOB medium (Sambrook et al, "Molecular Cloning A Laboratory Manual, Second Edition", Cold Spring Harbor Laboratory Press (1989)) with ampicillin and L-arabinose (1 mM). The obtained culture was grown with aeration at 30 °C to an OD₆₀₀ of ≈0.6 and then made electrocompetent by concentrating 100-fold and washing three times with ice-cold deionized H₂O. Electroporation was performed using 70 µl of a cell suspension and ≈100 ng of PCR product. After electroporation, cells were incubated with 1 ml of SOC medium (Sambrook et al, "Molecular Cloning A Laboratory Manual, Second Edition", Cold Spring Harbor Laboratory Press (1989)) at 37 °C for 2.5 h and then plated onto an L-agar medium containing 25 mg/l of chloramphenicol (Cm) and grown at 37 °C to select Cm^{R} recombinants. Then, to eliminate the pKD46 plasmid, 2 passages on L-agar medium with Cm at 42 °C were performed and the obtained colonies were tested for sensitivity to ampicillin.

### 2. Verification of nirB gene deletion by PCR.

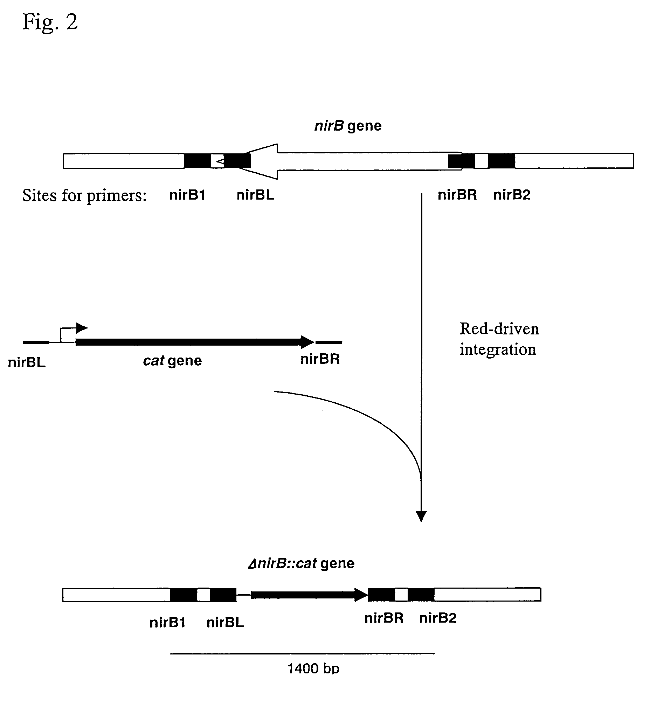

The mutants with the *nirB* gene deleted, marked with Cm resistance gene (*cat*)*,* were verified by PCR. Locus-specific primers nirB1 (SEQ ID NO: 4) and nirB2 (SEQ ID NO: 5) were used for verification by PCR. Conditions for PCR verification were as follows: denaturation step for 3 min at 94 °C; profile for the 30 cycles: 30 sec at 94 °C, 30 sec at 54 °C, 1 min at 72 °C; final step: 7 min at 72 °C. PCR product, obtained in the reaction with the cells of parental nirB⁺ strain MG1655 as a template, was 949 bp in length. The PCR product, obtained in the reaction with the cells of mutant MG1655 ΔnirB::cat strain as a template, was 1400 nucleotides in length (Fig.2).

### 3. Construction of arginine-producing strain with inactivated nirB gene.

The arginine-producing strain *E. coli* 237 (VKPM B-7925) was transduced to Cm resistance by the standard P1 transduction procedure (Sambrook et al, "Molecular Cloning A Laboratory Manual, Second Edition", Cold Spring Harbor Laboratory Press (1989)). The strain MG1655 ΔnirB::cat was used as a donor for *cat* gene. The resulting strain 237ΔnirB::cat was verified by PCR to have ΔnirB::cat deletion by means of primers nirB1 (SEQ ID NO: 4) and nirB2 (SEQ ID NO: 5).

### Example 2. Production of L-arginine by E. coli strain with inactivated nirB gene.

Both *E. coli* strains 237 and 237ΔnirB::cat were grown overnight at 37 °C on L-agar plates. The strain 237ΔnirB::cat plate also contained chloramphenicol (20 µg/ml). Then one loop of the cells was transferred to 2 ml of minimal medium for fermentation in the 20x200 mm test tubes. Cells were grown for 72 hours at 32 °C with shaking at 250 rpm.

After the cultivation, the amount of arginine which accumulated in the medium was determined by paper chromatography using arginine (1 g/l and 2 g/l) and glutamic acid (1 g/l and 2 g/l) as controls. The paper was developed with a mobile phase: n-butanol : acetic acid : water = 4 : 1 : 1 (v/v). A solution of ninhydrin (0.5%) in acetone was used as a visualizing reagent.

The results are presented in Table 1.

| The composition of the fermentation medium (g/l): | |
|---|---|
| Glucose | 67.0 |
| Yeast extract | 5.0 |
| (NH₄)₂SO₄ | 35.0 |
| KH₂PO₄ | 2.0 |
| MgSO₄•7H₂O | 2.0 |
| Thiamine (Vitamin B₁ ) | 1.0 |
| CaCO₃ | 25.0 |
| L-isoleucine | 0.05 |

Glucose and magnesium sulfate are sterilized separately. pH is adjusted to 7.2.

**Table 1.**

| *E. coli* strain | OD₅₅₅ | Arg (g/l) | Glu(g/l) |
|---|---|---|---|
| 237 | 21.1 | 5.6±0.5 | 4.6±0.5 |
| 237ΔnirB::cat | 19.9 | 6.8±0.6 | traces |

As it is seen from Table 1, inactivation of the nir operon improved the L-arginine accumulation by the L-arginine-producing strain 237.

## Claims

1. An L-amino acid-producing bacterium of the *Enterobacteriaceae* family, wherein the bacterium has been modified to inactivate the *nir* operon.

2. The L-amino acid-producing bacterium according to claim 1, wherein said *nir* operon comprises *nirBDC* and *cysG* genes.

3. The L-amino acid-producing bacterium according to claim 2, wherein said bacterium belongs to the genus *Escherichia.*

4. The L-amino acid-producing bacterium according to claim 1 , wherein said L-amino acid is L-arginine.

5. The L-amino acid-producing bacterium according to claim 4, wherein said bacterium has been modified to enhance expression of an L-arginine operon.

6. A method for producing an L-amino acid comprising:
- cultivating the bacterium according to claim 1 in a medium, and
- collecting the accumulated L-amino acid from the medium.

7. The method according to claim 6, wherein said L-amino acid is L-arginine.

8. The method according to claim 7, wherein said bacterium has been modified to enhance expression of an L-arginine operon.


SEQUENCE LISTING

<110> Ajinomoto Co., Inc.

<120> METHOD FOR PRODUCING L-AMINO ACID USING BACTERIUM
      OF ENTEROBACTERIACEAE FAMILY, HAVING nir OPERON
      INACTIVATED

<130> EPA-63519

<150> RU2003126289
<151> 2003-08-29

<160> 10

<170> PatentIn Ver. 2.1

<210> 1
<211> 61
<212> DNA
<213> Artificial Sequence

<220>
<223> Description of Artificial Sequence: primer

<400> 1
aatgagcaaa gtcagactcg caattatcgg taacggtatg gcgaataaat acctgtgacg 60
g                                                                 61

<210> 2
<211> 63
<212> DNA
<213> Artificial Sequence

<220>
<223> Description of Artificial Sequence: primer

<400> 2
ggctcatgcg ttgtcctcca ccagagttac tgggatacgt ttacgccccg ccctgccact 60
cat                                                               63

<210> 3
<211> 945
<212> DNA
<213> Artificial Sequence

<220>
<223> Description of Artificial Sequence: cat gene
      flanked by regions for integration

<400> 3
aatgagcaaa gtcagactcg caattatcgg taacggtatg gcgaatacct gtgacggaag 60
atcacttcgc agaataaata aatcctggtg tccctgttga taccgggaag ccctgggcca 120
acttttggcg aaaatgagac gttgatcggc acgtaagagg ttccaacttt caccataatg 180
aaataagatc actaccgggc gtattttttg agttatcgag attttcagga gctaaggaag 240
ctaaaatgga gaaaaaaatc actggatata ccaccgttga tatatcccaa tggcatcgta 300
aagaacattt tgaggcattt cagtcagttg ctcaatgtac ctataaccag accgttcagc 360
tggatattac ggccttttta aagaccgtaa agaaaaataa gcacaagttt tatccggcct 420
ttattcacat tcttgcccgc ctgatgaatg ctcatccgga attccgtatg gcaatgaaag 480
acggtgagct ggtgatatgg gatagtgttc acccttgtta caccgttttc catgagcaaa 540
ctgaaacgtt ttcatcgctc tggagtgaat accacgacga tttccggcag tttctacaca 600
tatattcgca agatgtggcg tgttacggtg aaaacctggc ctatttccct aaagggttta 660
ttgagaatat gtttttcgtc tcagccaatc cctgggtgag tttcaccagt tttgatttaa 720
acgtggccaa tatggacaac ttcttcgccc ccgttttcac catgggcaaa tattatacgc 780
aaggcgacaa ggtgctgatg ccgctggcga ttcaggttca tcatgccgtc tgtgatggct 840
tccatgtcgg cagaatgctt aatgaattac aacagtactg cgatgagtgg cagggcgggg 900
cgtaaacgta tcccagtaac tctggtggag gacaacgcat gagcc 945

<210> 4
<211> 24
<212> DNA
<213> Artificial Sequence

<220>
<223> Description of Artificial Sequence: primer

<400> 4
agccgtcacc gtcagcataa cacc 24

<210> 5
<211> 24
<212> DNA
<213> Artificial Sequence

<220>
<223> Description of Artificial Sequence: primer

<400> 5
gcacgctgga ctcgaagaac gggt 24

<210> 6
<211> 5476
<212> DNA
<213> Escherichia coli

<400> 6
   gtgacttaag aaaatttata caaatcagca atatacccat taaggagtat ataaaggtga 60
   atttgattta catcaataag cggggttgct gaatcgttaa ggtaggcggt aatagaaaag 120
   aaatcgaggc aaaaatgagc aaagtcagac tcgcaattat cggtaacggt atggtcggcc 180
   atcgctttat cgaagatctt cttgataaat ctgatgcggc caactttgat attaccgttt 240
   tctgtgaaga accgcgcatc gcttatgacc gcgtacacct ctcgtcttac ttctctcacc 300
   acaccgccga agagctgtcg ctggtgcgcg aaggcttcta cgagaaacac ggcatcaaag 360
   ttctggtcgg cgaacgcgct atcaccatca accgtcagga gaaggtgatt cactccagcg 420
   ccggacgtac cgttttttat gacaagctga tcatggcaac cggttcctac ccgtggatcc 480
   cgccaatcaa aggttctgat actcaggact gctttgtcta tcgcactatt gaagacctca 540
   acgccattga atcctgcgcc cgtcgcagca aacgcggtgc cgttgttggt ggcggcctgt 600
   taggtctgga agccgcaggc gcgctgaaaa acttaggtat tgaaacccac gttatcgaat 660
   ttgcccctat gctgatggca gaacagcttg atcagatggg cggcgagcag ctgcgtcgca 720
   aaatcgaaag tatgggcgtg cgcgttcaca ccagcaaaaa cacccttgag attgtgcagg 780
   aaggtgttga agcgcgtaaa accatgcgtt ttgccgacgg cagcgaactg gaagtcgact 840
   ttatcgtctt ctctaccggt atccgtccgc gcgataagct ggcaacccag tgtggtctgg 900
   acgttgctcc gcgtgggggt attgtcatta atgattcctg ccagacttcc gatccggata 960
   tctacgccat cggtgaatgc gcaagctgga acaaccgtgt atttggtctg gtagcacctg 1020
   gctacaaaat ggcgcaggtc gccgttgacc atattctcgg tagcgaaaac gcctttgaag 1080
   gtgctgacct tagcgccaag ctgaaactgc tgggcgtaga cgtaggcggt attggtgatg 1140
   cgcacggtcg cacgcctggc gcacgtagct acgtttacct cgacgaaagt aaagagatct 1200
   acaaacgcct gattgtcagc gaagacaaca aaaccctgct cggtgcggta ctggtgggcg 1260
   ataccagcga ctacggtaac ctgctgcaac tggtgctgaa cgctatcgaa ctgccggaaa 1320
   acccggattc cctgatcctg ccagcacact cgggtagcgg caagccgtct atcggtgttg 1380
   ataaactgcc ggacagcgcg caaatctgct cctgcttcga cgtcaccaaa ggtgatctga 1440
   ttgctgccat caacaaaggc tgccacacag ttgcggcgct gaaagctgaa accaaagcgg 1500
   gtactggctg cggtggctgt atcccgctgg tcactcaggt actgaacgcg gaactggcga 1560
   aacagggcat cgaagttaac aacaacctgt gcgaacactt tgcttattcg cgtcaggaac 1620
   tgttccattt gatccgcgtt gaaggcatta aaaccttcga agaactgctg gcgaaacacg 1680
   gcaaaggcta cggttgtgaa gtttgtaaac caaccgtcgg ttcgctgctg gcctcctgct 1740
   ggaacgaata cattctgaag ccggaacata ctccgctgca ggattctaac gacaacttcc 1800
   tcgctaacat ccagaaagac ggcacctact cggtgatccc gcgttctccg ggcggtgaaa 1860
   tcaccccgga agggctgatg gcggtaggtc gtatcgcgcg tgaatttaat ctctacacca 1920
   agatcactgg ctcccagcgt ctggcgatgt ttggcgcaca gaaagacgat ctgccggaga 1980
   tctggcgtca gctgattgaa gccggcttcg aaaccggtca tgcctatgcg aaagcactgc 2040
   gtatggcgaa aacctgcgtg ggtagcacct ggtgccgcta cggcgttggc gacagcgtcg 2100
   gcctcggcgt ggaactggaa aaccgctaca aaggcatccg tacgccgcac aaaatgaagt 2160
   tcggtgtctc cggctgtacc cgtgaatgtt cagaagctca gggtaaagac gtgggtatta 2220
   tcgccactga aaaaggctgg aacctgtatg tttgcggtaa cggcggcatg aaaccgcgtc 2280
   atgcggatct gctggcggcg gatatcgatc gcgaaacgct gatcaaatat ctcgaccgct 2340
   tcatgatgtt ctacatccgt actgccgaca aactgacgcg taccgcaccg tggttagaaa 2400
   acctcgaagg cggcatcgat tacctgaaag cagtgatcat tgacgacaaa ctggggctga 2460
   acgcacatct ggaagaagag atggcgcgcc tgcgtgaagc ggtactgtgt gagtggactg 2520
   aaacggtcaa tacgccgtct gcgcagactc gcttcaaaca cttcatcaac agcgacaagc 2580
   gtgacccgaa cgtgcagatg gtgccagagc gcgaacagca ccgtccggca acgccgtatg 2640
   aacgtatccc agtaactctg gtggaggaca acgcatgagc cagtggaaag acatctgcaa 2700
   aatcgatgac atcctgcctg aaaccggcgt ctgcgcgctg ttaggtgacg agcaggtcgc 2760
   gattttccgc ccgtatcaca gcgatcaggt gtttgcgatc agcaacatcg acccgttctt 2820
   cgagtccagc gtgctgtcac gcggactgat tgcggaacac cagggcgagc tgtgggtcgc 2880
   cagcccgctg aaaaaacagc gttttcgctt aagcgacggc ttgtgcatgg aagacgaaca 2940
   gttttccgtc aaacattacg aagcgcgagt gaaagacggc gtggtgcagc tgcgcggtta 3000
   atgttttaac gggaggcgca atgcctcccc tttttgcatg gtcctgtaat aatcttcggt 3060
   atattgcagg acatttttta aactttttgt tttatttttt gtttttattt tttaaaggat 3120
   aatcaaatgt ttacagacac tattaataag tgtgcggcta acgctgcgcg cattgcacgc 3180
   ctgtcggcaa ataacccgct cggcttttgg gtcagctccg ccatggcggg cgcgtatgtg 3240
   ggtcttggga tcatcctgat tttcacgctc ggtaatttgc tcgatccatc cgtacgccct 3300
   ttggtgatgg gcgcgacctt tggtatcgcc ttaacgctgg tgattatcgc cggttctgaa 3360
   ctgttcaccg gacacaccat gttcctcacc tttggggtaa aagcgggcag catcagccac 3420
   gggcaaatgt gggcaatcct gccgcaaacc tggctgggta acctggtcgg ttccgtcttc 3480
   gttgccatgc tctatagctg gggcggcggt agcctgctgc cggtagatac cagcatcgtt 3540
   cactccgtcg cgctggctaa aaccactgca ccggcaatgg tactcttctt caaaggtgca 3600
   ttgtgtaact ggctggtttg cctggcaatc tggatggcgc tgcgcactga aggggcggcg 3660
   aaatttatcg ctatctggtg gtgtctgctg gcatttatcg cgtccggcta cgagcactct 3720
   atcgctaaca tgacgctgtt cgcgctctcc tggttcggca accacagcga agcctacacg 3780
   ctggcgggta ttggtcataa cctgctgtgg gtgacgctgg gtaatacttt atcaggtgcc 3840
   gtattcatgg gattgggtta ttggtatgct acgccgaaag cgaatcgtcc ggttgcggac 3900
   aaatttaatc aaactgaaac ggctgccggt taattactaa ggggttttta cgtggatcat 3960
   ttgcctatat tttgccaatt acgcgatcgc gactgtctga ttgtcggcgg tggtgatgtc 4020
   gcggaacgca aagcaaggtt gctgttagac gcaggcgctc gcttaacggt gaatgcatta 4080
   gcgtttattc cacagttcac cgcatgggca gatgcaggca tgttaaccct cgtcgaaggg 4140
   ccatttgatg aaagccttct cgacacctgc tggctggcga ttgcagcgac ggatgatgac 4200
   gcgcttaacc agcgcgtcag cgaagccgct gaagctcgtc gcatcttctg taacgtggtc 4260
   gatgcgccga aagccgccag ctttattatg ccgtcgatta ttgaccgctc accgctcatg 4320
   gtagcggtct cctctggcgg cacctctccg gttctggcac gcctgttgcg cgaaaaactt 4380
   gaatcactgc tgccgttaca tctgggccag gtagcgaaat acgccgggca attacgcggg 4440
   cgagtgaaac aacagttcgc cacgatgggt gagcgtcgcc gtttctggga gaaattgttc 4500
   gttaacgacc gcctggcgca gtcgctggca aacaacgatc agaaagccat tactgaaacg 4560
   accgaacagt taatcaacga accgctcgac catcgcggtg aagtggtgct ggttggtgca 4620
   ggtccgggcg atgccgggct gctgacactg aaaggactgc aacaaattca gcaggcagat 4680
   gtggtggtct acgaccgtct ggtttctgac gatattatga atctggtacg ccgcgatgcg 4740
   gaccgtgttt tcgtcggcaa acgcgcggga taccactgcg taccccagga agagattaac 4800
   cagatcctgc tgcgggaagc gcaaaaaggc aaacgcgtgg tgcggctgaa aggtggcgat 4860
   ccgtttattt ttggccgtgg tggcgaagag ctggaaacac tgtgcaacgc gggtattccg 4920
   ttctcggtgg ttccgggtat taccgcagct tctggttgct ctgcctattc gggtattcca 4980
   ctcacgcatc gcgattatgc ccagagcgta cgcttaatta ccggacactt aaaaaccggt 5040
   ggcgagctgg actgggaaaa cctggcggca gaaaaacaga cgctggtgtt ctatatgggg 5100
   ttgaatcagg ccgcgactat tcagcaaaag ctgattgaac acggaatgcc aggcgaaatg 5160
   ccggtggcaa ttgtcgaaaa cggtacggca gtcacgcagc gcgtgattga cggtacgctc 5220
   acacagctgg gagaactggc gcagcaaatg aacagtccat cgctaattat tattggtcgg 5280
   gttgttggcc tgcgcgataa actgaactgg ttctccaacc attaatttaa cccgggccag 5340
   agaattctgg ccttcttaat gactcttttt atatattcag caaataaaac atagcccctt 5400
   aataaatatt atctgctaat gggttatatt caccgttaca gcgacttaca aaattaagtc 5460
   attcgctcac tgacgc 5476

<210> 7
<211> 847
<212> PRT
<213> Escherichia coli

<400> 7
Met Ser Lys Val Arg Leu Ala Ile Ile Gly Asn Gly Met Val Gly His
1               5                   10                  15

Arg Phe Ile Glu Asp Leu Leu Asp Lys Ser Asp Ala Ala Asn Phe Asp

            20                  25                  30

Ile Thr Val Phe Cys Glu Glu Pro Arg Ile Ala Tyr Asp Arg Val His
        35                  40                  45

Leu Ser Ser Tyr Phe Ser His His Thr Ala Glu Glu Leu Ser Leu Val
    50                  55                  60

Arg Glu Gly Phe Tyr Glu Lys His Gly Ile Lys Val Leu Val Gly Glu
65                  70                  75                  80

Arg Ala Ile Thr Ile Asn Arg Gln Glu Lys Val Ile His Ser Ser Ala
                85                  90                  95

Gly Arg Thr Val Phe Tyr Asp Lys Leu Ile Met Ala Thr Gly Ser Tyr
            100                 105                 110

Pro Trp Ile Pro Pro Ile Lys Gly Ser Asp Thr Gln Asp Cys Phe Val
        115                 120                 125

Tyr Arg Thr Ile Glu Asp Leu Asn Ala Ile Glu Ser Cys Ala Arg Arg
    130                 135                 140

Ser Lys Arg Gly Ala Val Val Gly Gly Gly Leu Leu Gly Leu Glu Ala
145                 150                 155                 160

Ala Gly Ala Leu Lys Asn Leu Gly Ile Glu Thr His Val Ile Glu Phe
                165                 170                 175

Ala Pro Met Leu Met Ala Glu Gln Leu Asp Gln Met Gly Gly Glu Gln
            180                 185                 190

Leu Arg Arg Lys Ile Glu Ser Met Gly Val Arg Val His Thr Ser Lys
        195                 200                 205

Asn Thr Leu Glu Ile Val Gln Glu Gly Val Glu Ala Arg Lys Thr Met
    210                 215                 220

Arg Phe Ala Asp Gly Ser Glu Leu Glu Val Asp Phe Ile Val Phe Ser
225                 230                 235                 240

Thr Gly Ile Arg Pro Arg Asp Lys Leu Ala Thr Gln Cys Gly Leu Asp
                245                 250                 255

Val Ala Pro Arg Gly Gly Ile Val Ile Asn Asp Ser Cys Gln Thr Ser
            260                 265                 270

Asp Pro Asp Ile Tyr Ala Ile Gly Glu Cys Ala Ser Trp Asn Asn Arg
        275                 280                 285

Val Phe Gly Leu Val Ala Pro Gly Tyr Lys Met Ala Gln Val Ala Val
    290                 295                 300

Asp His Ile Leu Gly Ser Glu Asn Ala Phe Glu Gly Ala Asp Leu Ser
305                 310                 315                 320

Ala Lys Leu Lys Leu Leu Gly Val Asp Val Gly Gly Ile Gly Asp Ala
                325                 330                 335

His Gly Arg Thr Pro Gly Ala Arg Ser Tyr Val Tyr Leu Asp Glu Ser
            340                 345                 350

Lys Glu Ile Tyr Lys Arg Leu Ile Val Ser Glu Asp Asn Lys Thr Leu
        355                 360                 365

Leu Gly Ala Val Leu Val Gly Asp Thr Ser Asp Tyr Gly Asn Leu Leu
    370                 375                 380

Gln Leu Val Leu Asn Ala Ile Glu Leu Pro Glu Asn Pro Asp Ser Leu
385                 390                 395                 400

Ile Leu Pro Ala His Ser Gly Ser Gly Lys Pro Ser Ile Gly Val Asp
                405                 410                 415

Lys Leu Pro Asp Ser Ala Gln Ile Cys Ser Cys Phe Asp Val Thr Lys
            420                 425                 430

Gly Asp Leu Ile Ala Ala Ile Asn Lys Gly Cys His Thr Val Ala Ala
        435                 440                 445

Leu Lys Ala Glu Thr Lys Ala Gly Thr Gly Cys Gly Gly Cys Ile Pro
    450                 455                 460

Leu Val Thr Gln Val Leu Asn Ala Glu Leu Ala Lys Gln Gly Ile Glu
465                 470                 475                 480

Val Asn Asn Asn Leu Cys Glu His Phe Ala Tyr Ser Arg Gln Glu Leu
                485                 490                 495

Phe His Leu Ile Arg Val Glu Gly Ile Lys Thr Phe Glu Glu Leu Leu
            500                 505                 510

Ala Lys His Gly Lys Gly Tyr Gly Cys Glu Val Cys Lys Pro Thr Val
        515                 520                 525

Gly Ser Leu Leu Ala Ser Cys Trp Asn Glu Tyr Ile Leu Lys Pro Glu
    530                 535                 540

His Thr Pro Leu Gln Asp Ser Asn Asp Asn Phe Leu Ala Asn Ile Gln
545                 550                 555                 560

Lys Asp Gly Thr Tyr Ser Val Ile Pro Arg Ser Pro Gly Gly Glu Ile
                565                 570                 575

Thr Pro Glu Gly Leu Met Ala Val Gly Arg Ile Ala Arg Glu Phe Asn
            580                 585                 590

Leu Tyr Thr Lys Ile Thr Gly Ser Gln Arg Leu Ala Met Phe Gly Ala
        595                 600                 605

Gln Lys Asp Asp Leu Pro Glu Ile Trp Arg Gln Leu Ile Glu Ala Gly
    610                 615                 620

Phe Glu Thr Gly His Ala Tyr Ala Lys Ala Leu Arg Met Ala Lys Thr
625                 630                 635                 640

Cys Val Gly Ser Thr Trp Cys Arg Tyr Gly Val Gly Asp Ser Val Gly
                645                 650                 655

Leu Gly Val Glu Leu Glu Asn Arg Tyr Lys Gly Ile Arg Thr Pro His
            660                 665                 670

Lys Met Lys Phe Gly Val Ser Gly Cys Thr Arg Glu Cys Ser Glu Ala

675                 680                 685

Gln Gly Lys Asp Val Gly Ile Ile Ala Thr Glu Lys Gly Trp Asn Leu
    690                 695                 700

Tyr Val Cys Gly Asn Gly Gly Met Lys Pro Arg His Ala Asp Leu Leu
705                 710                 715                 720

Ala Ala Asp Ile Asp Arg Glu Thr Leu Ile Lys Tyr Leu Asp Arg Phe
                725                 730                 735

Met Met Phe Tyr Ile Arg Thr Ala Asp Lys Leu Thr Arg Thr Ala Pro
            740                 745                 750

Trp Leu Glu Asn Leu Glu Gly Gly Ile Asp Tyr Leu Lys Ala Val Ile
        755                 760                 765

Ile Asp Asp Lys Leu Gly Leu Asn Ala His Leu Glu Glu Glu Met Ala
    770                 775                 780

Arg Leu Arg Glu Ala Val Leu Cys Glu Trp Thr Glu Thr Val Asn Thr
785                 790                 795                 800

Pro Ser Ala Gln Thr Arg Phe Lys His Phe Ile Asn Ser Asp Lys Arg
                805                 810                 815

Asp Pro Asn Val Gln Met Val Pro Glu Arg Glu Gln His Arg Pro Ala
            820                 825                 830

Thr Pro Tyr Glu Arg Ile Pro Val Thr Leu Val Glu Asp Asn Ala
        835                 840                 845

<210> 8
<211> 108
<212> PRT
<213> Escherichia coli

<400> 8
Met Ser Gln Trp Lys Asp Ile Cys Lys Ile Asp Asp Ile Leu Pro Glu
  1               5                  10                  15

Thr Gly Val Cys Ala Leu Leu Gly Asp Glu Gln Val Ala Ile Phe Arg
            20                  25                  30

Pro Tyr His Ser Asp Gln Val Phe Ala Ile Ser Asn Ile Asp Pro Phe
        35                  40                  45

Phe Glu Ser Ser Val Leu Ser Arg Gly Leu Ile Ala Glu His Gln Gly
    50                  55                  60

Glu Leu Trp Val Ala Ser Pro Leu Lys Lys Gln Arg Phe Arg Leu Ser
65                  70                  75                  80

Asp Gly Leu Cys Met Glu Asp Glu Gln Phe Ser Val Lys His Tyr Glu
                85                  90                  95

Ala Arg Val Lys Asp Gly Val Val Gln Leu Arg Gly
            100                 105


<210> 9
<211> 184
<212> PRT
<213> Escherichia coli

<400> 9
Met Phe Leu Thr Phe Gly Val Lys Ala Gly Ser Ile Ser His Gly Gln
1               5                   10                  15

Met Trp Ala Ile Leu Pro Gln Thr Trp Leu Gly Asn Leu Val Gly Ser
            20                  25                  30

Val Phe Val Ala Met Leu Tyr Ser Trp Gly Gly Gly Ser Leu Leu Pro
        35                  40                  45

Val Asp Thr Ser Ile Val His Ser Val Ala Leu Ala Lys Thr Thr Ala
    50                  55                  60

Pro Ala Met Val Leu Phe Phe Lys Gly Ala Leu Cys Asn Trp Leu Val
65                  70                  75                  80

Cys Leu Ala Ile Trp Met Ala Leu Arg Thr Glu Gly Ala Ala Lys Phe
                85                  90                  95

Ile Ala Ile Trp Trp Cys Leu Leu Ala Phe Ile Ala Ser Gly Tyr Glu
            100                 105                 110

His Ser Ile Ala Asn Met Thr Leu Phe Ala Leu Ser Trp Phe Gly Asn
        115                 120                 125

His Ser Glu Ala Tyr Thr Leu Ala Gly Ile Gly His Asn Leu Leu Trp
    130                 135                 140

Val Thr Leu Gly Asn Thr Leu Ser Gly Ala Val Phe Met Gly Leu Gly
145                 150                 155                 160

Tyr Trp Tyr Ala Thr Pro Lys Ala Asn Arg Pro Val Ala Asp Lys Phe
                165                 170                 175

Asn Gln Thr Glu Thr Ala Ala Gly
            180

<210> 10
<211> 457
<212> PRT
<213> Escherichia coli

<400> 10
Val Asp His Leu Pro Ile Phe Cys Gln Leu Arg Asp Arg Asp Cys Leu
1               5                   10                  15

Ile Val Gly Gly Gly Asp Val Ala Glu Arg Lys Ala Arg Leu Leu Leu
            20                  25                  30

Asp Ala Gly Ala Arg Leu Thr Val Asn Ala Leu Ala Phe Ile Pro Gln
        35                  40                  45

Phe Thr Ala Trp Ala Asp Ala Gly Met Leu Thr Leu Val Glu Gly Pro
    50                  55                  60

Phe Asp Glu Ser Leu Leu Asp Thr Cys Trp Leu Ala Ile Ala Ala Thr

65                  70                  75                  80

Asp Asp Asp Ala Leu Asn Gln Arg Val Ser Glu Ala Ala Glu Ala Arg
                85                  90                  95

Arg Ile Phe Cys Asn Val Val Asp Ala Pro Lys Ala Ala Ser Phe Ile
            100                 105                 110

Met Pro Ser Ile Ile Asp Arg Ser Pro Leu Met Val Ala Val Ser Ser
        115                 120                 125

Gly Gly Thr Ser Pro Val Leu Ala Arg Leu Leu Arg Glu Lys Leu Glu
    130                 135                 140

Ser Leu Leu Pro Leu His Leu Gly Gln Val Ala Lys Tyr Ala Gly Gln
145                 150                 155                 160

Leu Arg Gly Arg Val Lys Gln Gln Phe Ala Thr Met Gly Glu Arg Arg
                165                 170                 175

Arg Phe Trp Glu Lys Leu Phe Val Asn Asp Arg Leu Ala Gln Ser Leu
            180                 185                 190

Ala Asn Asn Asp Gln Lys Ala Ile Thr Glu Thr Thr Glu Gln Leu Ile
        195                 200                 205

Asn Glu Pro Leu Asp His Arg Gly Glu Val Val Leu Val Gly Ala Gly
    210                 215                 220

Pro Gly Asp Ala Gly Leu Leu Thr Leu Lys Gly Leu Gln Gln Ile Gln
225                 230                 235                 240

Gln Ala Asp Val Val Val Tyr Asp Arg Leu Val Ser Asp Asp Ile Met
                245                 250                 255

Asn Leu Val Arg Arg Asp Ala Asp Arg Val Phe Val Gly Lys Arg Ala
            260                 265                 270

Gly Tyr His Cys Val Pro Gln Glu Glu Ile Asn Gln Ile Leu Leu Arg
        275                 280                 285

Glu Ala Gln Lys Gly Lys Arg Val Val Arg Leu Lys Gly Gly Asp Pro
    290                 295                 300

Phe Ile Phe Gly Arg Gly Gly Glu Glu Leu Glu Thr Leu Cys Asn Ala
305                 310                 315                 320

Gly Ile Pro Phe Ser Val Val Pro Gly Ile Thr Ala Ala Ser Gly Cys
                325                 330                 335

Ser Ala Tyr Ser Gly Ile Pro Leu Thr His Arg Asp Tyr Ala Gln Ser
            340                 345                 350

Val Arg Leu Ile Thr Gly His Leu Lys Thr Gly Gly Glu Leu Asp Trp
        355                 360                 365

Glu Asn Leu Ala Ala Glu Lys Gln Thr Leu Val Phe Tyr Met Gly Leu
    370                 375                 380

Asn Gln Ala Ala Thr Ile Gln Gln Lys Leu Ile Glu His Gly Met Pro
385                 390                 395                 400

Gly Glu Met Pro Val Ala Ile Val Glu Asn Gly Thr Ala Val Thr Gln
                405                 410                 415

Arg Val Ile Asp Gly Thr Leu Thr Gln Leu Gly Glu Leu Ala Gln Gln
            420                 425                 430

Met Asn Ser Pro Ser Leu Ile Ile Ile Gly Arg Val Val Gly Leu Arg
        435                 440                 445

Asp Lys Leu Asn Trp Phe Ser Asn His
    450                 455